# EUROPEAN PATENT SPECIFICATION

(11) **EP 0 975 609 B1**
(45) Date of publication and mention of the grant of the patent: **27.10.2010**
(21) Application number: 98904853.3
(22) Date of filing: 02.02.1998
(51) Int. Cl.: C07D 265/18, A61K 9/14, B01D 9/00

(54) **PROCESS FOR THE CRYSTALLIZATION OF A REVERSE TRANSCRIPTASE INHIBITOR USING AN ANTI-SOLVENT**
VERFAHREN ZUR KRISTALLISATION EINES REVERSTRANSKRIPTASE-INHIBITORS, BEI WELCHEM EIN ANTI-LÖSUNGSMITTEL BENUTZT WIRD
PROCEDE DE CRISTALLISATION D'UN INHIBITEUR DE TRANSCRIPTASE INVERSE UTILISANT UN ANTISOLVANT

(30) Priority: 05.02.1997 US 37385 P; 24.03.1997 GB 9706046; 08.04.1997 US 42807 P; 07.05.1997 GB 9709348
(43) Date of publication of application: 02.02.2000
(73) Proprietor: Merck Sharp & Dohme Corp., Rahway, NJ 07065 (US)
(72) Inventor: CLARKE, William, Rahway, NJ 07065 (US); CROCKER, Louis, S., Rahway, NJ 07065 (US); KUKURA, Joseph, L., II, Rahway, NJ 07065 (US); THOMPSON, Andrew, S., Mountainside, NJ 07092 (US)
(74) Representative: Horgan, James Michael Frederic
(86) International application number: PCT/US1998/001999
(87) International publication number: WO 1998/033782

(56) References cited:
- EP-A- 0 169 618
- EP-A- 0 582 455
- WO-A-95/20389
- WO-A-98/45278
- US-A- 5 519 021
- US-A- 5 633 405
- US-A- 5 663 467
- THOMPSON A.S. ET AL.: "Use of an ephedrine alkoxide to mediate enantioselective addition of an acetylide to a prochiral ketone: Asymmetric synthesis of the reverse transcriptase inhibitor L-743,726" TETRAHEDRON LETTERS, NL, ELSEVIER SCIENCE PUBLISHERS, AMSTERDAM, vol. 36, no. 49, 4 December 1995 (1995-12-04), pages 8937-8940, XP004026777 ISSN: 0040-4039
- GIRON D.: "Thermal analysis and calorimetric methods in the characterisation of polymorphs and solvates" THERMOCHIMICA ACTA, ELSEVIER SCIENCE PUBLISHERS, AMSTERDAM, NL, vol. 248, 1995, pages 1-59, XP002101808 ISSN: 0040-6031
- THRELFALL T.L.: "Analysis of organic polymorphs. A review" ANALYST, LONDON, GB, vol. 120, October 1995 (1995-10), pages 2435-2460, XP002101807

## Description

### BACKGROUND OF THE INVENTION

The synthesis of the reverse transcriptase inhibitor (RTI), (-)-6-chloro-4-cyclopropylethynyl-4-trifluoromethyl-1,4-dihydro-2H-3,1-benzoxazin-2-one, also known as DMP-266 has been described in US Patent 5,519,021 issued on May 21, 1996 and the corresponding PCT International Patent Application WO 95/20389, which published on August 3, 1995. Additionally, the asymmetric synthesis of an enantiomeric benzoxazinone by a highly enantioselective acetylide addition and cyclization sequence has been described by Thompson, et al., Tetrahedron Letters 1995, 36, 937-940, as well as the PCT publication, WO 96/37457, which published on November 28, 1996.

The preparation of DMP-266 is also described in EP-A-0582455 (Merck & Co., Inc.) and Thompson et al., Tet. Lett., 36(49), 8937-8940, 1995. However, in none of these citations are the reproducible crystallisation methods of the present invention disclosed, nor are any crystalline forms of DMP-266 described.

The compound was previously crystallized from a heptane-tetrahydrofuran (THF) solvent system. The crystallization procedure required the use of high temperatures (about 90°C) to dissolve the final product. Crystals formed by nucleation during the cooling process. The crystals which were produced were Form II and are converted to the desired Form I while drying under vacuum at 90°C. This crystallization provided minimal purification and produced material with inconsistent physical properties. The final product slurry was extremely difficult to mix and handle due to its high viscosity and heterogeneous nature.

The instant invention describes a method for crystallizing (-)-6-chloro-4-cyclopropylethynyl-4-trifluoromethyl-1,4-dihydro-2H-3,1-benzoxazin-2-one from a solvent and anti-solvent solvent system and producing the crystalline product. The desired final crystal form. Form I, can be produced when using methanol or ethanol. Form II is isolated from 2-propanol and can be converted to the desired crystal form at low drying temperatures, as low at 40°C.

### BRIEF DESCRIPTION OF THE FIGURES

Figure 1.
   Flowsheet of the controlled anti-solvent addition crystallization method.
Figure 2.
   Flowsheet of the heel crystallization method.
Figure 3.
   X-ray powder diffraction pattern for Form I of (-)-6-chloro-4-cyclopropylethynyl-4-trifluoromethyl-1,4-dihydro-2H-3,1-benzoxazin-2-one,
Figure 4.
   X-ray powder diffraction pattern for Form II of (-)-6-chloro-4-cyclopropylethynyl-4-trifluoroinethyl-1,4-dihydro-2H-3,1-benzoxazin-2-one.
Figure 5.
   X-ray powder diffraction pattern for Form III of (-)-6-chloro-4-cyclopropylethylnyl-4-trifuoromethyl-1,4-dihydro-2H-3,1-benzoxazin-2-one.
Figure 6.
   DSC curve for Form III of (-)-6-chloro-4-cyclopropylethynyl-4-trifluoromethyl-1,4-dihydro-2H-3,1-benzoxazin-2-one.
Figure 7.
   TG analysis for Form III of (-)-6-chloro-4-cyclopropylethynyl-4-trifluoromethyl-1,4-dihydro-2H-3,1-benzoxazin-2-one.

### DETAILED DESCRIPTION OF THE INVENTION

A process for the crystallization of a compound of the structural formula 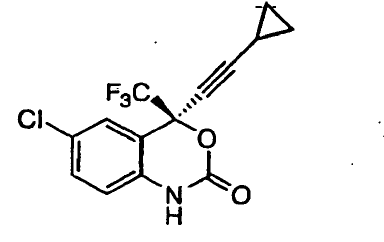 comprising the steps of:
(1) dissolving the compound in a solvent in a ratio of about 3.0 ml to about 10.0 ml of solvent to I gram of the compound;
(2) filtering the solution of the compound to remove any particulate matter;
(3) adding the anti-solvent to the stirring solution at room temperature over a period of about 30 minutes to about an hour to reach the saturation point of the solution containing the compound;
(4) adding to the solution a solid seed charge of Form I, Form II or Form III of the compound in the amount of about 2 to about 10 percent by weight to form a slurry;
(5) milling the slurry to reduce the thickness of the slurry;
(6) adding the remaining water to reach the desired solvent composition of about 30% to about 50% and milling the slurry as needed during the addition;
(7) slowly cooling the slurry to about 5°C to about 20°C;
(8) aging for about 2 to about 16 hours until the supernatant concentration reaches equilibrium;
(9) milling the slurry, as needed, to reduce the thickness of the slurry;
(10) filtering the milled slurry to isolate a wet cake of the crystalline compound;
(11) washing the wetcake once with about 1 to about 2 bed volumes of the final crystallization solvent composition and then twice with water using about 5-10 ml water per gram of compound; and
(12) drying the washed wetcake at about 40°C to about 90°C under vacuum for about 1 hour to about 3 days, or until the loss on dryness is less than 0.5 weight percent; to produce Form III when Form III seed and a solvent other than 2-propanol are used, otherwise to produce Form I.

The controlled anti-solvent crystallization process as recited above wherein the solvent is defined as alcohol, wherein alcohol is a straight or branched chain (C₁-C₆)-alkanol. A preferred embodiment of the solvents useful in the controlled anti-solvent crystallization process is (C₁-C₆)-alkanol, such as, methanol, ethanol, and 2-propanol. The preferred alcohol is 2-propanol.

The preferred alcohol is 2-propanol for treasons relating to obtaining consistent crystal forms. Although methanol and methanol solvent systems have been shown to be capable of producing the desired Form I crystal structure, a slight contamination of Form III crystals in a crystallization slurry in these systems can convert the entire slurry to containing exclusively Form III crystals, which are relatively difficult to convert to the desired Form I structure. Any known crystal structure of this compound placed in an about 25 % to 35 % (v/v) 2-propanol - water solvent system has been shown to quickly convert to the Form II crystal structure, which can readily convert to the desired Form I crystal structure during drying.

The anti-solvent as recited above is defined as a solvent in which the compound has limited solubility. In the instant process, the preferred anti-solvent is water.

The temperature of the solution during the anti-solvent addition (Step 3) was about 20°C to about 25°C. The temperature of the slurry being about 5°C to about 20°C, and preferrably at about 10°C.

The temperature used during the drying of the washed wetcake (Step 12) is about 40°C to about 90°C, and preferably about 40°C to about 60°C.

The solvent system (solvent plus anti-solvent) used ranged from about 30% to about 50% solvent volume to anti-solvent volume (v/v) ratio. The total volume of the solvent system ranging from about 12 to about 20 ml of the solvent system per gram of the compound. The solvent volume to anti-solvent volume ratio for selected solvent systems is as follows: 1) an ethanol-water solvent system is about 30% to about 40% ethanol to water v/v ratio; 2) a methanol-water solvent system is about 40% to about 50% methanol to water v/v ratio; and 3) an 2-propanol-water solvent system is about 25% to about 35% 2-propanol to water v/v ratio. The preferred solvent system is 2-propanol-water used in about a 30% volume to volume ratio and a total solvent system volume or about 15 ml per gram of the compound.

A process for the crystallization of a compound of the structural formula 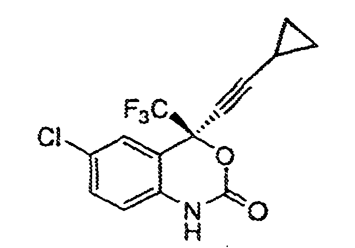 comprising the steps of:
(1) mixing about 10% to about 20% by weight of Form I, Form II or Form III of the final amount of (-)-6-chloro-4-cyclopropylethynyl-4-trifluoromethyl-1,4-dihydro-2H-3,1-benzoxazin-2-one in the desired v/v ratio of solvent to anti-solvent at about 20°C to form the heel or retaining a final slurry from a previous batch;
(2) adding the solution of solvent and (-)-6-chloro-4-cyclopropylethynyl-4-trifluomethyl-1,4-dihydro-2H-3,1-benzoxazin-2-one, and anti-solvent to the heel simultaneuosly at constant rates over about 6 hours maintaining the v/v ratio of solvent to anti-solvent;
(3) milling the slurry during the addition to reduce the thickness of the slurry;
(4) cooling the slurry to about 10°C over about 3 hours and aging slurry until the supernatant concentration reaches equilibrium;
(5) filtering the milled slurry to isolate a wet cake of the crystalline compound;
(6) washing the wetcake once with about 1 to about 2 bed volumes of the final crystallization solvent composition and then twice with water using about 5 ml to about 10 ml water per gram of compound; and
(7) drying the washed wetcake at about 40°C to about 90°C under vacuum for about 1 hour to about 3 days, or until the loss on dryness is less then 0.5 weight percent; to produce Form III when Form III is mixed and a solvent other than propan-2-ol is used, otherwise to obtain Form I.

The heel crystallization process as recited above wherein the solvent is defined as acetonitrile, dimethyl acetamide, dimethyl formamide or alcohol. A preferred embodiment of the solvents useful in the controlled anti-solvent crystallization process is alcohol, wherein alcohol is defined as (C₁-C₆)-alkanol, such as, methanol, ethanol, and 2-propanol. The preferred alcohol is 2-propanol.

The anti-solvent as recited above is defined as a solvent in which the compound has limited solubility. In the heel crystallization process the preferred anti-solvent is water.

The process as recited above wherein the temperature of the solution during the anti-solvent addition is about 5°C to about 20°C.

The temperature used during the drying of the washed wetcake is about 40°C to about 90°C, and preferrably about 40°C to about 60°C.

The solvent system (solvent plus anti-solvent) used ranges from about 30% to about 50% solvent volume to anti-solvent volume (v/v) ratio. The total volume of the solvent system ranging from about 12 to about 20 ml of the solvent system per gram of the compound. The solvent to anti-solvent volume to volume ratio for selected solvent systems is as follows: 1) an ethanol-water solvent system is about 30% to about 40% ethanol to water v/v ratio; 2) a methanol-water solvent system is about 40% to about 50% methanol to water v/v ratio; and 3) an 2-propanol-water solvent system is about 25% to about 35% 2-propanol to water v/v ratio. The perferred solvent system is 2-propanol-water used in about a 30% volume to volume ratio and a total solvent system volume of about 15 ml per gram of the compound.

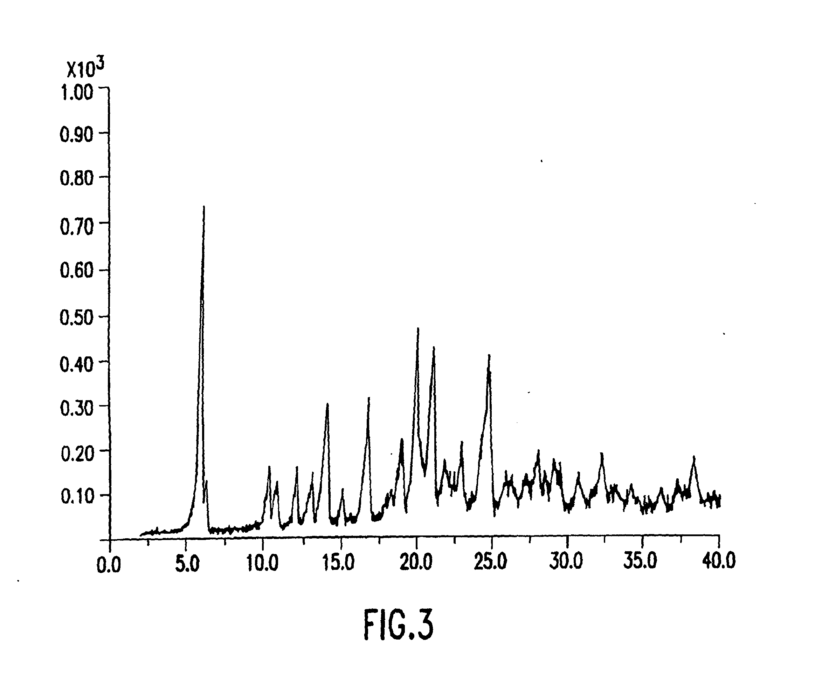

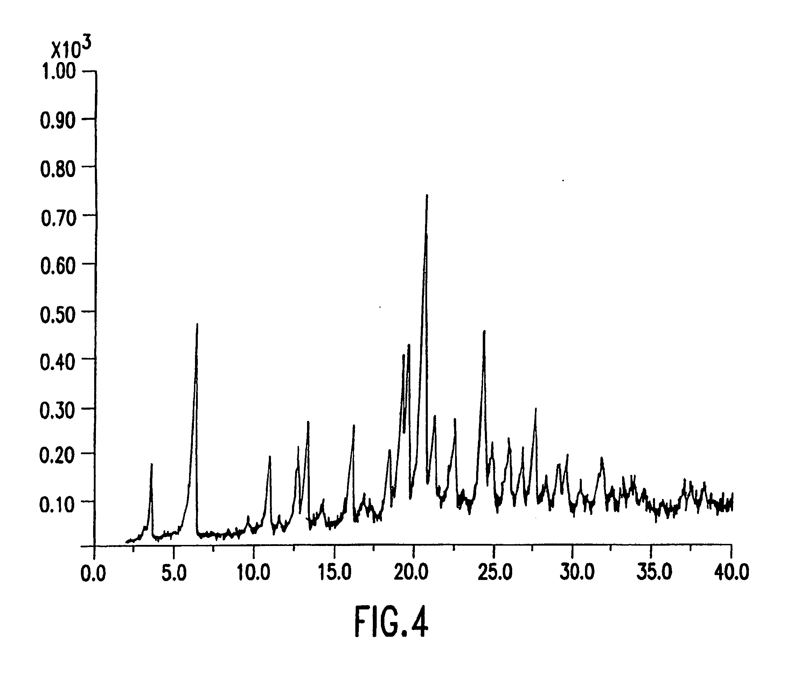

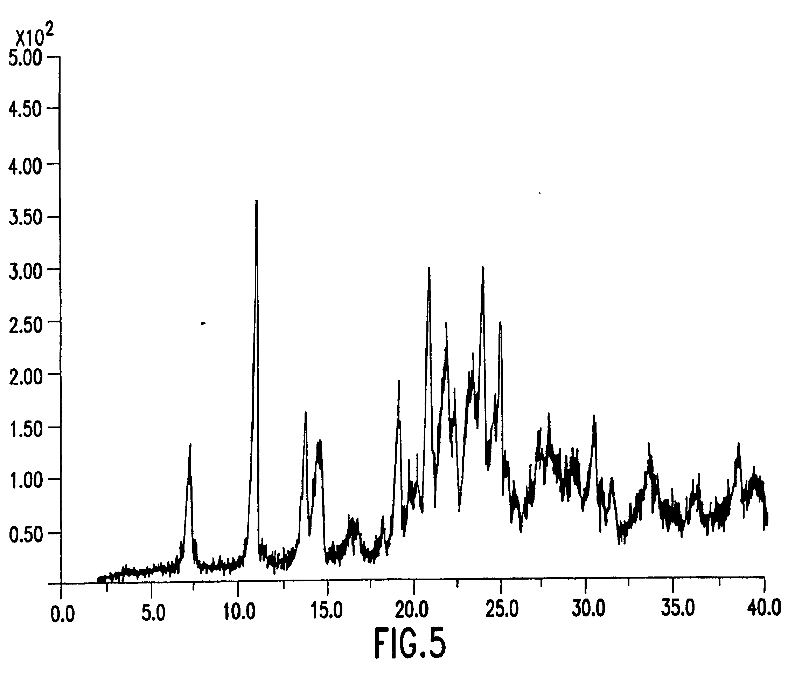

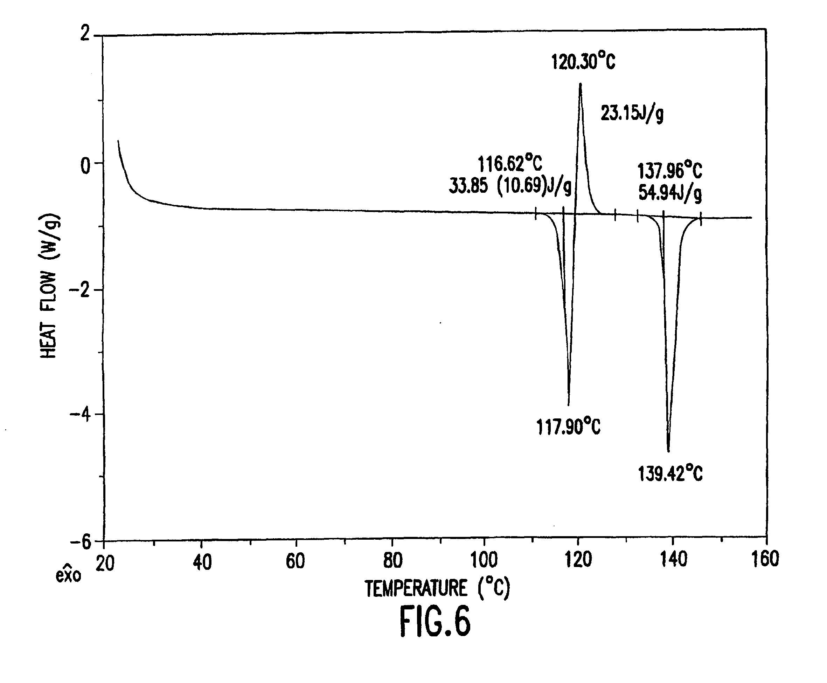

Figures 3, 4 and 5 are the X-ray powder diffraction (XRPD) patterns for Forms I, II and III, respectively. These XRPD patterns were recorded using an automated X-ray diffractometer APD 3720 with copper K alpha radiation. The crystal forms I and II of (-)-6-chloro-4-cyclopropylethynyl-4-trifluoromethyl-1,4-dihydro-2H-3,1-benzoxazin-2-one which are characterized by the noted X-ray powder diffraction patterns have the following key diffraction peaks (2Θ) with intensities (I/Iₘₐₓ, %) of 10 or greater:

| **Form I** | **Form II** | **Form III** |
|---|---|---|
| 6.0800 | 3.6375 | 7.2150 |
| 6.3900 | 6.3325 | 10.9675 |
| 10.3950 | 11.0725 | 13.7275 |
| 10.9875 | 12.7750 | 14.5325 |
| 12.2850 | 13.3275 | 16.7275 |
| 13.1900 | 14.2925 | 19.0675 |
| 14.1700 | 16.1200 | 19.6550 |
| 15.1925 | 16.8975 | 20.8250 |
| 16.9000 | 18.5025 | 21.7450 |
| 18.4375 | 19.1975 | 22.2825 |
| 19.2275 | 19.6025 | 22.8475 |
| 20.0925 | 20.6650 | 23.1750 |
| 21.2100 | 21.3250 | 23.8850 |
| 22.3600 | 22.6150 | 24.4900 |
| 23.0725 | 23.1775 | 24.9075 |
| 24.8900 | 24.4075 | 25.8200 |
| 25.9500 | 24.9650 | 27.0325 |
| 26.3575 | 26.0100 | 27.6050 |
| 27.2550 | 26.8550 | 29.2975 |
| 28.1150 | 27.6400 | 30.2600 |
| 28.5850 | 28.3675 | 30.7300 |
| 29.1325 | 29.1725 | 31.3125 |
| 29.5625 | 29.6325 | 33.3975 |
| 30.6850 | 30.5650 | 38.4325 |
| 32.3725 | 31.8950 | 39.2100 |
| 38.3125 | 33.8225 | |

Additionally, these crystal forms are characterized by peaks with varying D-spacings. Form I is characterized by peaks with D-spacings of: 14.5, 8.5, 8.0, 7.2, 6.7, 6.2, 5.2, 4.6, 4.4, 4.2, and 3.6 angstroms. Form II is characterized by peaks with D-spacings of: 24.3, 13.9, 8.0, 6.9, 6.6, 5.5, 4.6, 4.5, 4.3, 4.2, 3.9, 3.6, 3.4, 3.3, and 3.2 angstroms. Form III is characterized by peaks with D-spacings of: 12.2, 8.1, 6.4, 6.1, 4.7, 4.3, 4.1, 4.0, 3.9, 3.8, 3.7, 3.6, 3.3, 3.2, and 3.0 angstroms.

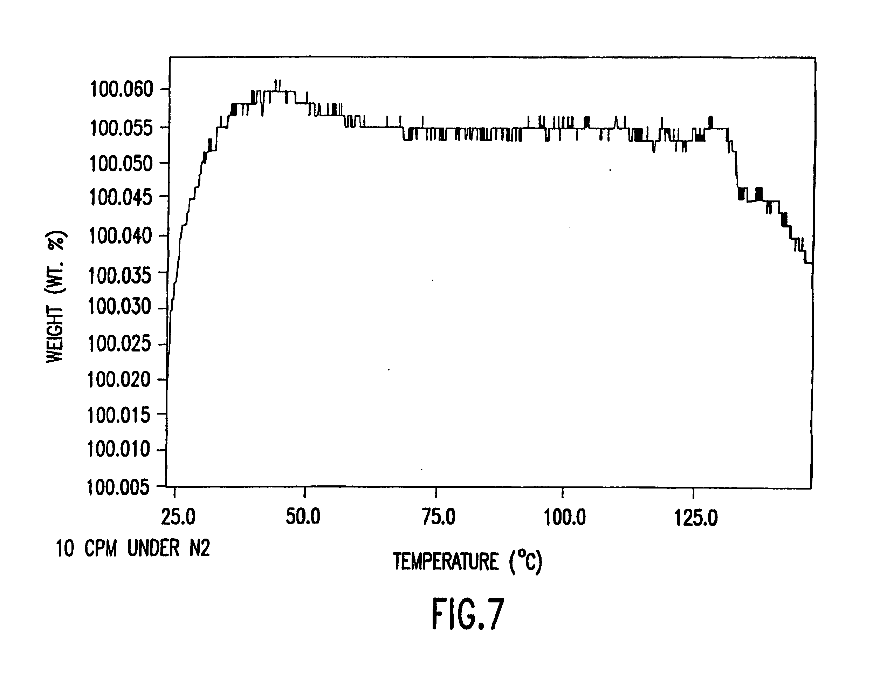

Thermogravimetric analysis results of Form III (Figure 7) indicated there was no significant weight loss observed from 43°C to about 137°C. This result is indicative of an anhydrous or unsolvated crystal form.

The differential scanning calorimetry (DSC) results obtained for Form III show an endotherm with an extrapolated onset temperature of 117°C, a peak temperature of 118°C, and an enthalpy of 34 J/g, this is followed by an exotherm with a peak temperature of 120°C and an enthalpy of 23J/g. A second endotherm with an extrapolated onset temperature of 138°C, a peak temperature of 139°C, and an enthalpy of 55 J/g is also observed. The first endotherm is associated with the melting of Form III, which subsequently crystallizes to Form I during the exothermic event. The second endothermic event is associated with the melting of Form I.

A process for isolating (-)-6-chloro-4-cyclopropylethynyl-4-trifluoromethyl-1,4-dihydro-2H-3,1-benzoxazin-2-one, the final product from solutions containing an organic solvent and water has been developed. In this process water serves as an anti-solvent to produce a solid product from material dissolved in organic solvent. The final solvent composition is chosen to balance yield loss, purification, and slurry handling properties.

For methanol solvent systems about 40% to about 50% volume to volume (v/v) solvent to anti-solvent (water) has been present in the final slurry. Ethanol solvent systems have contained about 30% to about 40% v/v solvent to anti-solvent, and 2-propanol solvent systems have used about 25% to about 35% (v/v) solvent to anti-solvent. The total amount of liquid (alcohol and water) ranges from 12 - 20 ml/(g solid). The crystallizations are typically performed between 20 - 25°C, and some slurries have been cooled to 5 - 10°C before being filtered. Following the filtration, the wetcake is washed with approximately one bed volume (the approximate volume of the wetcake) of the final crystallization solvent composition. The wetcake is then washed with at least 2 bed volumes of deionized (DI) water.

The rate at which the product is precipitated is controlled by either slowly adding the water to a saturated system following a seed charge (anti-solvent addition) or simultaneously adding the product in alcohol and water at controlled rates to an existing product slurry (heel crystallization).

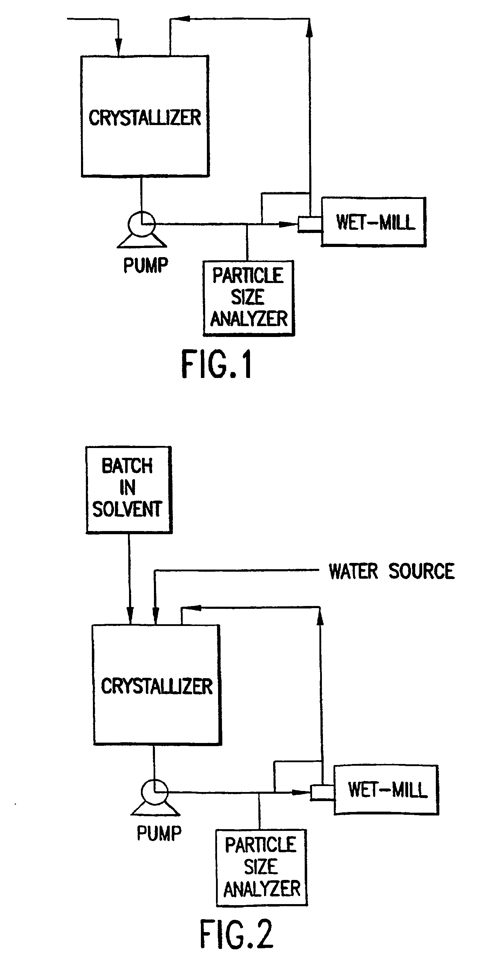

For the anti-solvent process (Figure 1), enough water is first added to the organic solvent solution containing the product over 0.5 - 2 hours to saturate the system in final product. A solid charge of final product is then added to the system as seed (2 - 10 % of the initial amount of product). The seed should be Form I (the crystal form associated with dry final product) for ethanol and methanol systems, and Form II seed (the crystal form generated from a THF/heptane crystallization) is used for 2-propanol systems. The resultant slurry is aged for 0.5 - 2 hours to establish a seed bed. The remaining water is then added over 2 - 4 hours in a controlled manner. The slurry is then aged for 2 - 20 hours and cooled to the desired final temperature during the age, allowing the supernatant to reach equilibrium.

For the heel process (Figure 2), a slurry at the desired final solvent composition is mixed while adding the product dissolved in alcohol and water at controlled relative rates to maintain a constant solvent ratio. The slurry (the crystalline compound in the final desired solvent system) is often 10-20% of the product from a prior run. The total charge is typically performed over 4 - 6 hours at 20 - 25°C. The slurry is then aged for several hours at the desired final temperature before being filtered to allow the supernatant to reach equilibrium. Following the filtration, the wetcake is washed with approximately one bed volume of a clean alcohol/water mixture matching the final crystallization conditions. The wetcake is then washed with at least 2 bed volumes of DI water.

Further control of the crystal size and slurry viscosity is achieved by using a wet-mill on slurries with excessively long particles and/or extremely thick consistencies. The product typically forms rod shaped crystals which grow much faster in the axial direction than the radial direction. It is understood that the reference to 'thickness' will refer to the crystal size and consistency of the slurry. The mill has been shown to reduce the length of the long crystals and produce a thin slurry from a thick slurry which contains many agglomerates of crystals. On the laboratory scale, the entire slurry can be milled batch-wise when desired. At larger scales, the wet-mill can be used on a recycle loop circulating around the crystallization vessel. An in-line particle size measurement and viscosity measurements could be coordinated to control the mill. Varying the temperature of the slurry through a range of 5°C to 50°C cycles has also been shown to be a useful way of modifying the crystal size and shape.

The solvents useful in this method include alcohol, acetonitrile (heel process only), dimethyl formamide (heel process only), and dimethyl acetamide (heel process only), The preferred solvent is an alcohol selected form methanol, ethanol or 2-propanol.

This crystallization process is advantageous over the prior method. The instant method allows one to isolate a crystalline product with consistent physical properties namely the ability to produce the desired crystal form of the product or convert to Form I with mild drying conditions (heating to about 40 to 60°C). The alcohol-water crystallizations have also been shown to reject some impurities carried forward from the chemical synthesis. The final product slurry is less viscous and more homogenous with the instant process and is thus easier to mix and handle.

The following examples are meant to be illustrative of the present invention. These examples are presented to exemplify the invention and are not to be construed as limiting the scope of the invention.

### EXAMPLE 1

### Controlled Anti-Solvent Addition Crystallization Process

400 g of DMP-266 starting material is dissolved in 2.400 L of ethanol. See Figure 1. The solution is filtered to remove extraneous matter. 2.088 L of deionized (DI) water is added to the solution over 30 to 60 minutes. 20 g of DMP-266 seed is added to the solution. The seed bed is aged for 1 hour. The use of Intermig agitators is preferred to mix the slurry. If required (by the presence of extremely long crystals or a thick slurry), the slurry is wet-milled for 15 - 60 seconds. 1.512 L of DI water is added to the slurry over 4 to 6 hours. If required (by the presence of extremely long crystals or a thick slurry), the slurry is wet-milled for 15 to about 60 seconds during the addition. The slurry is aged for 1 to 3 hours before being cooled to 10°C over 3 hours. The slurry is aged for 2 to 16 hours until the product concentration in the supernatant remains constant. The slurry is filtered to isolate a crystalline wet cake. The wet cake is washed with 1 to 2 bed volumes of 40 % ethanol in water and then twice with 2 L of DI water each. The washed wet cake is dried under vacuum at 50°C.

### EXAMPLE 2

### Semi-Continuous Heel Crystallization Process

400 g of DMP-266 starting material is dissolved in 2.400 L of ethanol. See Figure 2. A heel slurry is produced by mixing 20 g of DMP-266 in 0.3 L of 40 % (v/v) ethanol in water. The dissolved batch and 3.6 L of DI water are simultaneously charged to the heel slurry at constant rates over 6 hours to maintain a constant solvent composition in the crystallizer. Use of Intermig agitators during the crystallization is preferred. During this addition the slurry is wet-milled when the crystal lengths become excessively long or the slurry becomes too thick. The slurry is cooled to about 10°C over 3 hours. The slurry is aged for 2 to 16 hours until the product concentration in the supernatant remains constant. The slurry is filtered to isolate a crystalline wet cake. The wet cake is washed with 1 to 2 bed volumes of 40 % ethanol in water and then twice with 2 L of DI water each. The washed wet cake is dried under vacuum at 50°C.

### EXAMPLES 3-8

Following the crystallization procedures described in Examples 1 and 2 above using the solvents noted in the table below in the amounts recited DMP-266 can be crystallized.

| Ex #: Solvent | ml solvent per g. DMP-266* | ml H₂O per g. DMP-266* | Anti-solvent Process | Heel Process |
|---|---|---|---|---|
| EX. 3: Acetonitrile | 3.6 - 8.0 | 7.2 - 14 | - | X |
| EX. 4: Dimethyl acetamide | 3.6 - 8.0 | 7.2 - 14 | - | X |
| EX. 5: Dimethyl formamide | 3.6 - 8.0 | 7.2 - 14 | - | X |
| EX. 6: Ethanol | 3.6 - 8.0 | 7.2 - 14 | X | X |
| EX. 7: Methanol | 4.8 - 10 | 5.4 - 12 | X | X |
| EX. 8: 2-Propanol | 3.0 - 7.0 | 7.8 - 15 | X | X |

| | | | | |
|---|---|---|---|---|
| * The sum of the amounts of solvent and water should be at least 12 ml/g. The currently preferred concentration is 15 ml/g. - method did not work. X method worked. | | | | |

### EXAMPLE 9

### Crystallization of DMP-266 from 30% 2-Propanol in Water using a ratio of 15 ml solvent per gram DMP-266 Using Controlled Anti-Solvent Addition on a 400 g Scale

400 g. of DMP-266 starting material is dissolved in 1.8 L of 2-propanol. The solution is filtered to remove extraneous matter. 1.95 L of deionized (DI) water is added to the solution over 30 to 60 minutes. 10 g. to 20 g. of DMP-266 seed (Form II wetcake) is added to the solution. The seed bed is aged for 1 hour. The use of Intermig agitators is preferred to mix the slurry. If required (by the presence of extremely long crystals or a thick slurry), the slurry is wet-milled for 15-60 seconds. 2.25 L of DI water is added to the slurry over 4 to 6 hours. If required (by the presence of extremely long crystals or a thick slurry), the slurry is wet-milled for 15 - 60 seconds during the addition. The slurry is aged for 2 to 16 hours until the product concentration in the supernatant remains constant. The slurry is filtered to isolate a crystalline wet cake. The wet cake is washed with 1 to 2 bed volumes of 30 % 2-propanol in water and then twice with 1 bed volume of DI water each. The washed wet cake is dried under vacuum at 50 °C.

### EXAMPLE 10

### Crystallization of DMP-266 from 30% 2-Propanol in Water using a ratio of 15 ml solvent per gram DMP-266 Using a Semi-Continuous Process on a 400 g Scale

400 g. of DMP-266 starting material is dissolved in 1.8 L of 2-propanol. A heel slurry is produced by mixing 20 g. of Form II DMP-266 in 0.3 L of 30 % (v/v) 2-propanol in water or retaining part of a slurry froma previous crystallization in the crystallizer. The dissolved batch and 4.2 L of DI water are simultaneously charged to the heel slurry at constant rates over 6 hours to maintain a constant solvent composition in the crystallizer. Use of Intermig agitators during the crystallization is preferred. During this addition the slurry is wet-milled when the crystal lengths become excessively long or the slurry becomes too thick. The slurry is aged for 2 to 16 hours until the product concentration in the supernatant remains constant. The slurry is filtered to isolate a crystalline wet cake. The wet cake is washed with 1 to 2 bed volumes of 30 % 2-propanol in water and then twice with 1 bed volume of DI water each. The washed wet cake is dried under vacuum at 50 °C.

## Claims

1. A process for the crystallization of a compound of the structural formula: 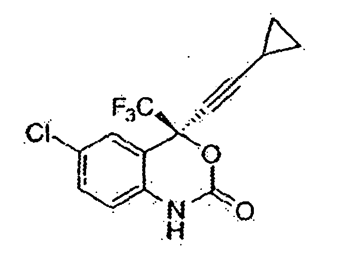 comprising the steps of:
(1) dissolving the compound in a solvent in a ratio of about 3.0 ml to about 10.0 ml of solvent to 1 gram of the compound;
(2) filtering the solution of the compound to remove any particulate matter;
(3) adding an anti-solvent to the stirring solution at room temperature over a period of about 30 minutes to about an hour to reach the saturation point of the solution containing the compound;
(4) adding to the solution a solid seed charge of Form I, Form II or Form III of the compound in the amount of about 2 to about 10 percent by weight to form a slurry, wherein Form I is
**characterized by** an X-ray powder diffraction pattern having crystallographic D-spacings of 14.5, 8.5, 8.0, 7.2, 6.7, 6.2, 5.2, 4.6, 4.4, 4.2 and 3.6 Angstroms, Form II is
**characterized by** an X-ray powder diffraction pattern having crystallographic D-spacings of 24.3, 13.9, 8.0, 6.9, 6.6, 5.5, 4.6, 4.5, 4.3, 4.2, 3.9, 3.6, 3.4, 3.3 and 3.2 Angstroms, and Form III
is **characterized by** an X-ray powder diffraction pattern having crystallographic D-spacings of 12.2, 8.1, 6.4, 6.1, 4.7, 4.3, 4.1, 4.0, 3.9, 3.8, 3.7, 3.6, 3.3, 3.2 and 3.0 Angstroms;
(5) milling the slurry to reduce the thickness of the slurry;
(6) adding the remaining anti-solvent to reach the desired solvent composition of about 30% to about 50% and milling the slurry as needed during the addition;
(7) slowly cooling the slurry to about 5°C to about 20°C;
(8) ageing for about 2 to about 16 hours until the supernatant concentration reaches equilibrium;
(9) milling the slurry, as needled, to reduce the thickness of the slurry;
(10) filtering the milled slurry to isolate a wet cake of the crystalline compound;
(11) washing the wet cake once with about 1 to about 2 bed volumes of the final crystallization solvent composition and then twice with water using about 5-10 ml water per gram of compound; and
(12) drying the washed wet cake at about 40°C to about 90°C under vacuum for about 1 hour to about 3 days, or until the loss on dryness is less than 0.5 weight percent; to produce Form III when Form III seed and a solvent other than 2-propanol are used, otherwise to produce Form I.

2. The process as recited in Claim 1 wherein the solvent is defined as (C₁-C₆)-alcohol.

3. The process as recited in Claim 2 wherein the anti-solvent is defined as water.

4. The process as recited in Claim 1 wherein the temperature during the anti-solvent addition (Step 3) is about 20°C to about 25°C.

5. The process as recited in Claim 2 wherein the temperature used during the drying of the washed wet cake (Step 12) is about 40°C to abut 60°C.

6. The process as recited in Claim 3 wherein the solvent volume to anti-sulvent volume ratio used is about 30% to about 50%.

7. The process as recited in Claim 6 wherein the (C₁-C₆)-alcohol is selected from methanol, ethanol and 2-propanol.

8. The process as recited in Claim 7 wherein the (C₁-C₆)-alcohol is 2-propanol.

9. The process as recited in Claim 7 wherein the total solvent system volume per gram of compound is about 12 ml/g to about 20 ml/g of about a 30% to about a 40% ethanol to water solvent to anti-solvent volume to volume ratio.

10. The process as recited in Claim 6 wherein the total solvent system volume per gram of compound is about 12 ml/g to about 20 ml/g of about 40% to about 50% methanol to water solvent to anti-solvent volume to volume ratio.

11. The process as recited in Claim 3 wherein the total solvent system volume per gram of compound is a haut 12 ml/g to about 20 ml/g of about 25% to about 35% 2-propanol to water solvent to anti-solvent volume to volume ratio.

12. The process as recited in Claim 8 wherein the total solvent system volume per gram of compound is about 15 ml/g of about 30% 2-propanol to water solvent to anti-solvent volume to volume ratio.

13. A process for the crystallization of a compound of the structural formula: 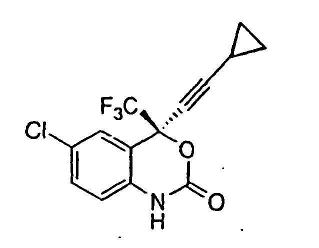 comprising the steps of:
(1) mixing about 10% to about 20% by weight of Form I, Form II or Form III of the final amount of (-)-6-chloro-4-cyclopropylethynyl-4-trifluoromethy-1,4-dihydro-2H-3,1-benzoxazin-2-one in the desired v/v ratio of solvent to anti-solvent at about 20°C to form a heel;
(2) adding the solution of solvent and (-)-6-chloro-4-cyclopropylethynyl-4-trifluoromethyl-1,4-dihydro-2H-3,1-benzoxazin-2-one, and anti-solvent to the heel simultaneously at constant rates over about 6 hours maintaining the v/v ratio of solvent to anti-solvent;
(3) milling the slurry during the addition to reduce the thickness of the slurry;
(4) cooling the slurry to about 10°C over about 3 hours and ageing slurry until the supernatant concentration reaches equilibrium;
(5) filtering the milled slurry to isolate a wet cake of the crystalline compound;
(6) washing the wet cake once with about 1 to about 2 bed volumes of the final crystallization solvent composition and then twice with water using about 5 ml to about 10 ml water per gram of compound; and
(7) drying the washed wet cake at about 40°C to about 90°C under vacuum for about 1 hour to about 3 days, or until the loss on dryness is less then 0.5 weight percent; to produce Form III when Forum III is mixed and a solvent other than propan-2-ol is used, otherwise to obtain Form I.

14. The process is recited in Claim 13 wherein solvent is defined as acetonitrile, dimethyl acetamide, dimethyl formamide or alcohol.

15. The process as recited in Claim 14 wherein the anti-solvent is defined as water.

16. The process as recited in Claim 15 wherein the alcohol solvent is defined as (C₁-C₆)-alcohol.

17. The process as recited in Claim 14 wherein the temperature of the solution during the compound-solvent solution/anti-solvent addition (Step 2) is about 5°C to about 20°C.

18. The process as recited in Claim 14 wherein the temperature used during the drying of the washed wet cake (Seep 7) .is about 40°C to about 60°C.

19. The process as recited in Claim 16 wherein the alcohol to anti-solvent volume to volume ratio used is about 30% to about 50%.

20. The process as recited in Claim 19 wherein the alcohol solvent is selected from methanol, methanol and 2-propanoi.

21. The process as recited in Claim 20 wherein the solvent is 2-propanol.

22. The process as recited in Claim 20 wherein the total solvent system volume per gram of compound is about 12 ml/g to about 20 ml/g of about a 30% to about a 40% ethanol to water solvent to anti-solvent volume to volume ratio.

23. The process as recited in Claim 20 wherein the total solvent system volume per gram of compound is about 12 ml/g to about 20 ml/g of about 40% to about 50% methanol to water solvent to anti-solvent volume to volume ratio.

24. The process as recited in Claim 21 wherein the total solvent system volume per gram of compound is about 12 ml/g to about 20 ml/g of about 25% to about 35% 2-propanol to water solvent to anti-solvent volume to volume ratio.

25. The process as recited in Claim 21 wherein the total solvent system volume per gram of compound is about 15 ml/g of about 30% 2-propanol to water solvent to anti-aolvent volume to volume ratio.

26. A processor the crystallization of Form II of (-)-6-chloro-4-cyclopropylethynyl-4-trifluoromethyl-1,4-dihydro-2H-3,1-benzoxazin-2-one, which comprises:
dissolving (-)-6-chloro-4-cyclopropylethnyl-4-trifluoromethyl-1,4-dihydro-2H-3,1-benzoxazin-2-one in 2-propanol;
adding water to obtain a 25% to 35% 2-propanol to water solvent to anti-solvent volume to volume ratio to initiate crystallization; and
isolating the crystallise Form II.

27. A process for obtaining crystalline Form I of (-)-6-chloro-4-cyclopropylethynyl-4-trifluoromethyl-1,4-dihydro-2H-3,1-benzoxazin-2-one, which comprises:
dissolving (-)-6-chloro-4-cyclopropylethynyl-4-trifluoromethyl-1,4-dihydro-2H-3,1-benzoxazin-2-one in 2-propanol;
adding water to obtain a 25% to 35% 2-propanol to water solvent to anti-solvent volume to volume ratio to initiate crystallization;
isolating the crystalline Form II; and
converting Form II to Form I by drying.

## Patentansprüche

1. Ein Verfahren zur Kristallisation einer Verbindung der Strukturformel: 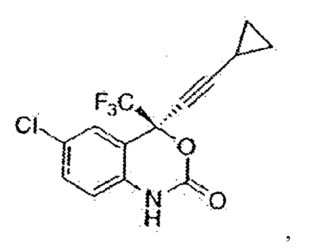 umfassend die Schritte:
(1) Auflösen der Verbindung in einem Lösungsmittel in einem Verhältnis von etwa 3,0 ml bis etwa 10,0 ml Lösungsmittel zu 1 Gramm der Verbindung,
(2) Filtrieren der Lösung der Verbindung, um etwaiges teilchenförmiges Material zu entfernen,
(3) Zugeben eines Antilösungsmittels zu der gerührten Lösung bei Raumtemperatur innerhalb eines Zeitraums von etwa 30 Minuten bis etwa 1 Stunde, um den Sättigungspunkt der Lösung, welche die Verbindung enthält, zu erreichen,
(4) Zugeben einer festen Impfcharge der Form I, Form II oder Form III der Verbindung in der Menge von etwa 2 bis etwa 10 Gewichtsprozent zu der Lösung, um eine Aufschlämmung zu bilden, wobei Form I **gekennzeichnet ist durch** ein Röntgenpulverbeugungsdiagramm mit Kristall-D-Abständen von 14,5, 8,5, 8,0, 7,2, 6,7, 6,2, 5,2, 4,6, 4,4, 4,2 und 3,6 Angström, Form II **gekennzeichnet ist durch** ein Röntgenpulverbeugungsdiagramm mit Kristall-D-Abständen von 24,3, 13,9, 8,0, 6,9, 6,6, 5,5, 4,6, 4,5, 4,3, 4,2, 3,9, 3,6, 3,4, 3,3 und 3,2 Angström, und Form III **gekennzeichnet ist durch** ein Röntgenpulverbeugungsdiagramm mit Kristall-D-Abständen von 12,2, 8,1, 6,4, 6,1, 4,7, 4,3, 4,1, 4,0, 3,9, 3,8, 3,7, 3,6, 3,3, 3,2 und 3,0 Angström,
(5) Mahlen der Aufschlämmung, um die Dicke der Aufschlämmung zu verringern,
(6) Zugeben des restlichen Antilösungsmittels, um die erwünschte Lösungsmittelzusammensetzung von etwa 30% bis etwa 50% zu ergeben, und Mahlen der Aufschlämmung nach Bedarf während des Zugebens,
(7) langsames Abkühlen der Aufschlämmung auf etwa 5°C bis etwa 20°C,
(8) Altern für etwa 2 bis etwa 16 Stunden, bis die Konzentration des Überstands ein Gleichgewicht erreicht,
(9) Mahlen der Aufschlämmung nach Bedarf, um die Dicke der Aufschlämmung zu verringern,
(10) Filtrieren der gemahlenen Aufschlämmung, um einen nassen Filterkuchen der kristallinen Verbindung zu isolieren,
(11) Waschen des nassen Filterkuchens einmal mit etwa 1 bis etwa 2 Bettvolumen der finalen Kristallisationslösungsmittelzusammensetzung und anschließend zweimal mit Wasser unter Verwendung von etwa 5-10 ml Wasser pro Gramm Verbindung, und
(12) Trocknen des gewaschenen nassen Filterkuchens bei etwa 40°C bis etwa 90°C unter Vakuum für etwa 1 Stunde bis etwa 3 Tage oder bis der Trockenverlust weniger als 0,5 Gewichtsprozent beträgt,
um Form III zu bilden, falls Form-III-Impfkeim und ein Lösungsmittel anders als 2-Propanol verwendet werden, ansonsten um Form I zu bilden.

2. Das Verfahren gemäß Anspruch 1, wobei das Lösungsmittel definiert ist als (C₁-C₆)-Alkohol.

3. Das Verfahren gemäß Anspruch 2, wobei das Antilösungsmittel definiert ist als Wasser.

4. Das Verfahren gemäß Anspruch 1, wobei die Temperatur während der Zugabe von Antilösungsmittel (Schritt 3) etwa 20°C bis etwa 25°C beträgt.

5. Das Verfahren gemäß Anspruch 2, wobei die während des Trocknens des gewaschenen nassen Filterkuchens (Schritt 12) verwendete Temperatur etwa 40°C bis etwa 60°C beträgt.

6. Das Verfahren gemäß Anspruch 3, wobei das verwendete Verhältnis von Lösungsmittelvolumen zu Antilösungsmittelvolumen etwa 30% bis etwa 50% beträgt.

7. Das Verfahren gemäß Anspruch 6, wobei der (C₁-C₆)-Alkohol ausgewählt ist aus Methanol, Ethanol und 2-Propanol.

8. Das Verfahren gemäß Anspruch 7, wobei der (C₁-C₆)-Alkohol 2-Propanol ist.

9. Das Verfahren gemäß Anspruch 7, wobei das Gesamtlösungsmittelsystemvolumen pro Gramm Verbindung etwa 12 ml/g bis etwa 20 ml/g eines Ethanol-zu-Wasser-Lösungsmittel-zu-Antilösungsmittel-Volumen-zu-Volumen-Verhältnisses von etwa 30% bis etwa 40% beträgt.

10. Das Verfahren gemäß Anspruch 6, wobei das Gesamtlösungsmittelsystemvolumen pro Gramm Verbindung etwa 12 ml/g bis etwa 20 ml/g eines Methanol-zu-Wasser-Lösungsmittel-zu-Antilösungsmittel-Volumen-zu-Volumen-Verhältnisses von etwa 40% bis etwa 50% beträgt.

11. Das Verfahren gemäß Anspruch 3, wobei das Gesamtlösungsmittelsystemvolumen pro Gramm Verbindung etwa 12 ml/g bis etwa 20 ml/g eines 2-Propanol-zu-Wasser-Lösungsmittel-zu-Antilösungsmittel-Volumen-zu-Volumen-Verhältnisses von etwa 25% bis etwa 35% beträgt.

12. Das Verfahren gemäß Anspruch 8, wobei das Gesamtlösungsmittelsystemvolumen pro Gramm Verbindung etwa 15 ml/g eines 2-Propanol-zu-Wasser-Lösungsmittel-zu-AntilösungsmittelVolumen-zu-Volumen-Verhältnisses von etwa 30% beträgt.

13. Ein Verfahren zur Kristallisation einer Verbindung der Strukturformel: 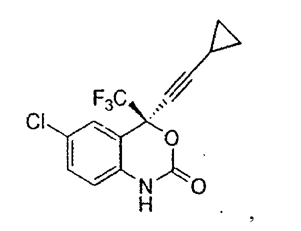 umfassend die Schritte:
(1) Vermischen von etwa 10 Gew.-% bis etwa 20 Gew.-% der Form I, Form II oder Form III der finalen Menge von (-)-6-Chlor-4-cyclopropylethinyl-4-trifluormethyl-1,4-dihydro-2H-3,1-benzoxazin-2-on in dem erwünschten Vol./Vol.-Verhältnis von Lösungsmittel zu Antilösungsmittel bei etwa 20°C, um einen Heel zu bilden,
(2) Zugeben der Lösung aus Lösungsmittel und (-)-6-Chlor-4-cyclopropylethinyl-4-trifluormethyl-1,4-dihydro-2H-3,1-benzoxazin-2-on und von Antilösungsmittel gleichzeitig zu dem Heel mit konstanten Geschwindigkeiten innerhalb von etwa 6 Stunden, wobei das Vol./Vol.-Verhältnis von Lösungsmittel zu Antilösungsmittel beibehalten wird,
(3) Mahlen der Aufschlämmung während der Zugabe, um die Dicke der Aufschlämmung zu verringern,
(4) Abkühlen der Aufschlämmung auf etwa 10°C innerhalb etwa 3 Stunden und Altem der Aufschlämmung, bis die Konzentration des Überstands ein Gleichgewicht erreicht,
(5) Filtrieren der gemahlenen Aufschlämmung, um einen nassen Filterkuchen der kristallinen Verbindung zu isolieren,
(6) Waschen des nassen Filterkuchens einmal mit etwa 1 bis etwa 2 Bettvolumen der finalen Kristallisationslösungsmittelzusammensetzung und anschließend zweimal mit Wasser unter Verwendung von etwa 5 ml bis etwa 10 ml Wasser pro Gramm Verbindung, und
(7) Trocknen des gewaschenen nassen Filterkuchens bei etwa 40°C bis etwa 90°C unter Vakuum für etwa 1 Stunde bis etwa 3 Tage oder bis der Trockenverlust weniger als 0,5 Gewichtsprozent beträgt,
um Form III zu erzeugen, falls Form III vermischt wird und ein Lösungsmittel anders als Propan-2-ol verwendet wird, ansonsten um Form I zu erhalten.

14. Das Verfahren gemäß Anspruch 13, wobei das Lösungsmittel definiert ist als Acetonitril, Dimethylacetamid, Dimethylformamid oder Alkohol.

15. Das Verfahren gemäß Anspruch 14, wobei das Antilösungsmittel definiert ist als Wasser.

16. Das Verfahren gemäß Anspruch 15, wobei das Alkohollösungsmittel definiert ist als (C₁-C₆)-Alkohol.

17. Das Verfahren gemäß Anspruch 14, wobei die Temperatur der Lösung während der Zugabe der Verbindung-Lösungsmittel-Lösung/Antilösungsmittel (Schritt 2) etwa 5°C bis etwa 20°C beträgt.

18. Das Verfahren gemäß Anspruch 14, wobei die während des Trocknens des gewaschenen nassen Filterkuchens (Schritt 7) verwendete Temperatur etwa 40°C bis etwa 60°C beträgt.

19. Das Verfahren gemäß Anspruch 16, wobei das verwendete Volumen-zu-VolumenVerhältnis von Alkohol zu Antilösungsmittel etwa 30% bis etwa 50% beträgt.

20. Das Verfahren gemäß Anspruch 19, wobei das Alkohollösungsmittel ausgewählt ist aus Methanol, Ethanol und 2-Propanol

21. Das Verfahren gemäß Anspruch 20, wobei das Lösungsmittel 2-Propanol ist.

22. Das Verfahren gemäß Anspruch 20, wobei das Gesamtlösungsmittelsystemvolumen pro Gramm Verbindung etwa 12 ml/g bis etwa 20 ml/g eines Ethanol-zu-Wasser-Lösungsmittel-zu-Antilösungsmittel-Volumen-zu-Volumen-Verhältnisses von etwa 30% bis etwa 40% beträgt.

23. Das Verfahren gemäß Anspruch 20, wobei das Gesamtlösungsmittelsystemvolumen pro Gramm Verbindung etwa 12 ml/g bis etwa 20 ml/g eines Methanol-zu-Wasser-Lösungsmittel-zu-Antilösungsmittel-Volumen-zu-Volumen-Verhältnisses von etwa 40% bis etwa 50% beträgt.

24. Das Verfahren gemäß Anspruch 21, wobei das Gesamtlösungsmittelsystemvolumen pro Gramm Verbindung etwa 12 ml/g bis etwa 20 ml/g eines 2-Propanol-zu-Wasser-Lösungsmittel-zu-Antilösungsmittel-Volumen-zu-Volumen-Verhältnisses von etwa 25% bis etwa 35% beträgt.

25. Das Verfahren gemäß Anspruch 21, wobei das Gesamtlösungsmittelsystemvolumen pro Gramm Verbindung etwa 15 ml/g eines 2-Propanol-zu-Wasser-Lösungsmittel-zu-AntilösungsmittelVolumen-zu-Volumen-Verhältnisses von etwa 30% beträgt.

26. Ein Verfahren zur Kristallisation der Form II von (-)-6-Chlor-4-cyclopropylethinyl-4-trifluormethyl-1,4-dihydro-2H-3,1-benzoxazin-2-on, umfassend:
Auflösen von (-)-6-Chlor-4-cyclopropylethinyl-4-trifluormethyl-1,4-dihydro-2H-3,1-benzoxazin-2-on in 2-Propanol,
Zugeben von Wasser, um ein 2-Propanol-zu-Wasser-Lösungsmittel-zu-AntilösungsmittelVolumen-zu-Volumen-Verhältnis von 25% bis 35% zu ergeben und die Kristallisation zu initiieren, und
Isolieren der kristallinen Form II.

27. Ein Verfahren zur Gewinnung der kristallinen Form I von (-)-6-Chlor-4-cyclopropylethinyl-4-trifluormethyl-1,4-dihydro-2H-3,1-benzoxazin-2-on, umfassend:
Auflösen von (-)-6-Chlor-4-cyclopropylethinyl-4-trifluormethyl-1,4-dihydro-2H-3,1-benzoxazin-2-on in 2-Propanol,
Zugeben von Wasser, um ein 2-Propanol-zu-Wasser-Lösungsmittel-zu-AntilösungsmittelVolumen-zu-Volumen-Verhältnis von 25% bis 35% zu ergeben und die Kristallisation zu initiieren,
Isolieren der kristallinen Form II und
Umwandeln von Form II in Form I durch Trocknen.

## Revendications

1. Procédé pour la cristallisation d'un composé de la formule structurale: 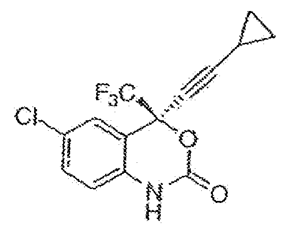 comprenant les étapes de:
(1) dissolution du composé dans un solvant en un rapport d'environ 3,0 ml à environ 10,0 ml de solvant pour 1 gramme du composé;
(2) filtration de la solution du composé pour retirer toute matière particulaire;
(3) ajout d'un anti-solvant à la solution sous agitation à la température ambiante sur une période d'environ 30 minutes à environ une heure pour atteindre le point de saturation de la solution contenant le composé;
(4) ajout à la solution d'une charge de germe solide de la Forme I, de la Forme II ou de la Forme III du composé dans la quantité d'environ 2 à environ 10 pour-cent en poids pour former une suspension épaisse, dans lequel la Forme I est **caractérisée par** un diagramme de diffraction des rayons X de poudre avec des espacements cristallographiques D de 14,5, 8,5, 8,0, 7,2, 6,7, 6,2, 5,2, 4,6, 4,4, 4,2 et 3,6 Angströms, la Forme II est **caractérisée par** un diagramme de diffraction des rayons X de poudre avec des espacements cristallographiques D de 24,3, 13,9, 8,0, 6,9, 6,6, 5,5, 4,6, 4,5, 4,3, 4,2, 3,9, 3,6, 3,4, 3,3 et 3,2 Angströms, et la Forme III est **caractérisée par** un diagramme de diffraction des rayons X de poudre avec des espacements cristallographiques D de 12,2, 8,1, 6,4, 6,1, 4,7, 4,3, 4,1, 4,0, 3,9, 3,8, 3,7, 3,6, 3,3, 3,2 et 3,0 Angströms;
(5) broyage de la suspension épaisse pour réduire l'épaisseur de la suspension épaisse;
(6) ajout de l'anti-solvant restant pour atteindre la composition de solvant désirée d'environ 30% à environ 50% et broyage de la suspension épaisse suivant les besoins durant l'ajout;
(7) refroidissement lent de la suspension épaisse jusqu'à environ 5°C à environ 20°C;
(8) vieillissement pendant environ 2 à environ 16 heures jusqu'à ce que la concentration du surnageant atteigne l'équilibre;
(9) broyage de la suspension épaisse, suivant les besoins, pour réduire l'épaisseur de la suspension épaisse;
(10) filtration de la suspension épaisse broyée pour isoler un gâteau humide du composé cristallin;
(11) lavage du gâteau humide une fois avec environ 1 à environ 2 volumes de lit de la composition de solvant de cristallisation finale et ensuite deux fois avec de l'eau en utilisant environ 5-10 ml d'eau par gramme de composé; et
(12) séchage du gâteau humide lavé à environ 40°C à environ 90°C sous vide pendant environ 1 heure à environ 3 jours, ou jusqu'à ce que la perte lors du séchage soit de moins de 0,5 pour-cent en poids;
pour produire la Forme III lorsqu'un germe de Forme III et un solvant autre que le 2-propanol sont utilisés, autrement pour produire la Forme I.

2. Procédé selon la revendication 1, dans lequel il est défini que le solvant est un alcool (C₁-C₆).

3. Procédé selon la revendication 2, dans lequel il est défini que l'anti-solvant est l'eau.

4. Procédé selon la revendication 1, dans lequel la température durant l'ajout de l'anti-solvant (étape 3) est d'environ 20°C à environ 25°C.

5. Procédé selon la revendication 2, dans lequel la température utilisée durant le séchage du gâteau humide lavé (étape 12) est d'environ 40°C à environ 60°C.

6. Procédé selon la revendication 3, dans lequel le rapport du volume du solvant sur le volume de l'anti-solvant utilisé est d'environ 30% à environ 50%.

7. Procédé selon la revendication 6, dans lequel l'alcool (C₁-C₆) est choisi parmi le méthanol, l'éthanol et le 2-propanol.

8. Procédé selon la revendication 7, dans lequel l'alcool (C₁-C₆) est le 2-propanol.

9. Procédé selon la revendication 8, dans lequel le volume du système de solvant total par gramme de composé est d'environ 12 ml/g à environ 20 ml/g d'un rapport de volume sur volume de solvant sur anti-solvant d'éthanol sur eau d'environ 30% à environ 40%.

10. Procédé selon la revendication 6, dans lequel le volume du système de solvant total par gramme de composé est d'environ 12 ml/g à environ 20 ml/g d'un rapport de volume sur volume de solvant sur anti-solvant de méthanol sur eau d'environ 40% à environ 50%.

11. Procédé selon la revendication 6, dans lequel le volume du système de solvant total par gramme de composé est d'environ 12 ml/g à environ 20 ml/g d'un rapport de volume sur volume de solvant sur anti-solvant de 2-propanol sur eau d'environ 25% à environ 35%.

12. Procédé selon la revendication 10, dans lequel le volume du système de solvant total par gramme de composé est d'environ 15 ml/g d'un rapport de volume sur volume de solvant sur anti-solvant de 2-propanol sur eau d'environ 30%.

13. Procédé pour la cristallisation d'un composé de la formule structurale: 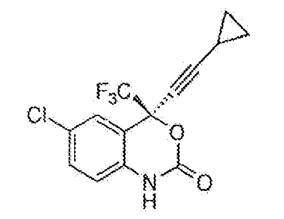 comprenant les étapes de:
(1) mélange d'environ 10% à environ 20% en poids de la Forme I, de la Forme II ou de la Forme III de la quantité finale de (-)-6-chloro-4-cyclopropyléthynyl-4-trifluorométhyl-1,4-dihydro-2H-3,1-benzoxazin-2-one dans le rapport v/v désiré de solvant sur anti-solvant à environ 20°C pour former un produit de queue;
(2) ajout de la solution de solvant et de (-)-6-chloro-4-cyclopropyléthynyl-4-trifluorométhyl-1,4-dihydro-2H-3,1-benzoxazin-2-one, et de l'anti-solvant au produit de queue simultanément à des vitesses constantes sur environ 6 heures en maintenant le rapport v/v de solvant sur anti-solvant;
(3) broyage de la suspension épaisse durant l'ajout pour réduire l'épaisseur de la suspension épaisse;
(4) refroidissement de la suspension épaisse jusqu'à environ 10°C sur environ 3 heures et vieillissement de la suspension épaisse jusqu'à ce que la concentration du surnageant atteigne l' équilibre;
(5) filtration de la suspension épaisse broyée pour isoler un gâteau humide du composé cristallin;
(6) lavage du gâteau humide une fois avec environ 1 à environ 2 volumes de lit de la composition de solvant de cristallisation finale et ensuite deux fois avec de l'eau en utilisant environ 5 à environ 10 ml d'eau par gramme de composé; et
(7) séchage du gâteau humide lavé à environ 40°C à environ 90°C sous vide pendant environ 1 heure à environ 3 jours, ou jusqu'à ce que la perte lors du séchage soit de moins de 0,5 pour-cent en poids;
pour produire la Forme III lorsque la Forme III est mélangée et un solvant autre que le propan-2-ol est utilisé, autrement pour obtenir la Forme I.

14. Procédé selon la revendication 13, dans lequel il est défini que le solvant est l'acétonitrile, le diméthylacétamide, le diméthylformamide ou un alcool.

15. Procédé selon la revendication 14, dans lequel il est défini que l'anti-solvant est l'eau.

16. Procédé selon la revendication 15, dans lequel il est défini que le solvant alcool est un alcool (C₁-C₆).

17. Procédé selon la revendication 14, dans lequel la température de la solution durant l'ajout de la solution de solvant-composé/anti-solvant (étape 2) est d'environ 5°C à environ 20°C.

18. Procédé selon la revendication 14, dans lequel la température utilisée durant le séchage du gâteau humide lavé (étape 7) est d'environ 40°C à environ 60°C.

19. Procédé selon la revendication 16, dans lequel le rapport de volume sur volume de l'alcool sur l'anti-solvant utilisé est d'environ 30% à environ 50%.

20. Procédé selon la revendication 17, dans lequel le solvant alcool est choisi parmi le méthanol, l'éthanol et le 2-propanol.

21. Procédé selon la revendication 20, dans lequel le solvant est le 2-propanol.

22. Procédé selon la revendication 20, dans lequel le volume du système de solvant total par gramme de composé est d'environ 12 ml/g à environ 20 ml/g d'un rapport de volume sur volume de solvant sur anti-solvant d'éthanol sur eau d'environ 30% à environ 40%.

23. Procédé selon la revendication 20, dans lequel le volume du système de solvant total par gramme de composé est d'environ 12 ml/g à environ 20 ml/g d'un rapport de volume sur volume de solvant sur anti-solvant de méthanol sur eau d'environ 40% à environ 50%.

24. Procédé selon la revendication 21, dans lequel le volume du système de solvant total par gramme de composé est d'environ 12 ml/g à environ 20 ml/g d'un rapport de volume sur volume de solvant sur anti-solvant de 2-propanol sur eau d'environ 25% à environ 35%.

25. Procédé selon la revendication 23, dans lequel le volume du système de solvant total par gramme de composé est d'environ 15 ml/g d'un rapport de volume sur volume de solvant sur anti-solvant de 2-propanol sur eau d'environ 30%.

26. Procédé pour la cristallisation de la Forme II de (-)-6-chloro-4-cyclopropyléthynyl-4-trifluorométhyl-1,4-dihydro-2H-3,1-benzoxazin-2-one, qui comprend:
la dissolution de la (-)-6-chloro-4-cyclopropyléthynyl-4-trifluorométhyl-1,4-dihydro-2H-3,1-benzoxazin-2-one dans du 2-propanol;
l'ajout d'eau pour obtenir un rapport de volume sur volume de solvant sur anti-solvant de 2-propanol sur eau de 25% à 35% pour initier une cristallisation; et
l'isolement de la Forme cristalline II.

27. Procédé pour l'obtention de la Forme cristalline I de (-)-6-chloro-4-cyclopropyléthynyl-4-trifluorométhyl-1,4-dihydro-2H-3,1-benzoxazin-2-one, qui comprend:
la dissolution de la (-)-6-chloro-4-cyclopropyléthynyl-4-trifluorométhyl-1,4-dihydro-2H-3,1-benzoxazin-2-one dans du 2-propanol;
l'ajout d'eau pour obtenir un rapport de volume sur volume de solvant sur anti-solvant de 2-propanol sur eau de 25% à 35% pour initier une cristallisation;
l'isolement de la Forme cristalline II; et
la conversion de la Forme II en Forme I par un séchage.
